# EUROPÄISCHE PATENTSCHRIFT

(11) **EP 1 382 325 B1**
(45) Veröffentlichungstag und Bekanntmachung des Hinweises auf die Patenterteilung: **02.11.2006**
(21) Anmeldenummer: 03016028.7
(22) Anmeldetag: 15.07.2003
(51) Int. Cl.: A61K 8/26, A61K 8/73, A61Q 15/00

(54) **Verfahren zur Herstellung einer desodorierenden Zubereitung, desodorierende Zubereitung und deren Verwendung**
Process for making a deodorant composition, deodorant composition and use thereof
Procédé de préparation d'une composition déodorante, la composition déodorante et son utilisation

(30) Priorität: 19.07.2002 DE 20210904 U
(43) Veröffentlichungstag der Anmeldung: 21.01.2004
(73) Patentinhaber: Holzlehner, Martin, 30175 Hannover (DE); Holzlehner, Frank, 30625 Hannover (DE)
(72) Erfinder: Weiland-Groterjahn, Heinz-Jürgen, 31789 Hameln (DE); Holzlehner, Martin, 30175 Hannover (DE); Holzlehner, Frank, 30625 Hannover (DE)
(74) Vertreter: Körner, Peter

(56) Entgegenhaltungen:
- BE-A- 1 007 311
- DE-A- 4 419 962
- US-A- 5 260 053
- US-A- 6 139 824
- US-A1- 2001 006 653
- J.S. JELLINEK: "Formulation and function of cosmetics" 1970 , WILEY XP002261035 * Seite 300 *
- DATABASE CHEMICAL ABSTRACTS [Online] retrieved from STN Database accession no. 96: 129 598 XP002261037 & JP 56 045436 A (OSAKA AEROSOL INDUSTRY CO., LTD) 25. April 1981 (1981-04-25)
- DATABASE WPI [Online] DERWENT PUBLICATIONS LTD., LONDON, GB; Database accession no. 1988-194566 (25) XP002261038 & JP 63 132661 A (NIPPON ZEON KK) 4. Juni 1988 (1988-06-04)
- R. WINTER: "a consumer's dictionary of cosmetic ingredients" 1999 , THREE RIVERS PRESS , US XP002261036 277720 * Seite 51 *
- DATABASE CHEMICAL ABSTRACTS [Online] STN Retrieved from STN Database accession no. 109: 115 420

## Beschreibung

Die Erfindung betrifft ein Verfahren zur Herstellung einer desodorierenden Zubereitung, eine desodorierende Zubereitung und deren Verwendung.

Im Bereich der Körperpflege werden Desodorantien zur Beseitigung störender Körpergerüche eingesetzt. Diese entstehen bei der bakteriellen Zersetzung des an sich geruchlosen Schweißes, insbesondere in den feuchtwarmen Achselhöhlen oder unter ähnlichen, den Mikroorganismen gute Lebensbedingungen bietenden Bedingungen.

Zur Vermeidung derartiger Körpergerüche ist die Verwendung von Alaun-Kristallen, nachfolgend als Deo-Kristalle bezeichnet, bekannt.

Zur Anwendung werden diese Deo-Kristalle angefeuchtet, wodurch sich an der Kristalloberfläche ein dünner Film einer wässrigen Lösung dieses Kristalls ausbildet, welcher dann auf die Haut aufgetragen wird. Die Körperwärme lässt das aufgetragene Wasser verdunsten und der Wirkstoff bildet feinste Depots auf der Haut. Er unterstützt die Haut bei der Bildung des natürlichen Säureschutzmantels und verhindert so das Entstehen von geruchsbildenden Bakterien. Einmal täglich angewendet, schützt ein Deo-Kristall üblicherweise mindestens 12 Stunden lang vor Körpergeruch. Derartige Deo-Kristalle sind vorteilhaft frei von chlorierten Aluminiumsalzen, verursachen kein Verstopfen der Schweißdrüsen, wirken nur auf der Haut und reduzieren nicht die natürliche Transpiration.

Bekannte Deo-Kristalle bestehen aus Kaliumalaun und/oder Ammoniumalaun. Die Herstellung von Kaliumalaun erfolgt beispielsweise durch Zusammengeben der wässrigen Lösungen von K₂SO₄ und Al₂(SO₄)₃ und anschließendem Auskristallisieren. Die Herstellung von Ammoniumalaun erfolgt entsprechend durch Zusammengeben von (NH₄)₂SO₄ und Al₂(SO₄)₃. Der Ausgangsstoff Al₂(SO₄)₃ ist beispielsweise aus H₂SO₄ und Al(OH)₃ herstellbar.

Um Deo-Kristalle von besonderer Reinheit zu erhalten, wird ein Alaun vorteilhaft mehrfach kristallisiert, vorzugsweise bis zu 12 mal in Wasser gelöst und wieder auskristallisiert.

Die hergestellten Deo-Kristalle wirken sehr gut desodorierend und/oder desinfizierend, bereiten jedoch Schwierigkeiten bei der Handhabung: Die Kristalle sind zerbrechlich und können beispielsweise beim Fallenlassen zerstört werden. Außerdem ist das Anfeuchten des Deo-Kristalls und das Auftragen des Flüssigkeitsfilms auf die Haut umständlich.

Um die Handhabung zu verbessern, ist aus der DE 44 19 962 A1 ein Deo-Zerstäuber bekannt, mit der eine einfache Nutzung von desodorierenden und/oder desinfizierenden Deo-Kristallen möglich sein soll. Hierzu enthält der Deo-Zerstäuber eine gesättigte Lösung von desodorierenden und/oder desinfizierenden Salzen oder Doppelsalzen und einen ungelösten Anteil dieser Salze oder Doppelsalze, wobei die Salze oder Doppelsalze zumindest aus Kalium, Aluminium und Sulfat oder zumindest aus Ammonium, Aluminium und Sulfat, jeweils in Ionenform, bestehen. Bevorzugt wird als Salz oder Doppelsalz ein Alaun, insbesondere ein Ammoniumalaun, verwendet.

Nachteilig an der in der DE 44 19 962 A1 vorgeschlagenen desodorierenden Zubereitung, die mittels eines Deo-Zerstäubers auf die Haut aufgesprüht wird, ist jedoch, dass die Düse des Deo-Zerstäubers aufgrund auskristallisierender Salze schnell verstopft. Darüber hinaus hat sich als Nachteil erwiesen, dass die auf den Körper aufgesprühte desodorierende Zubereitung derart wässrig ist, dass sie am Körper verläuft. Es ist auch bekannt, dass eine derartige desodorierende Zubereitung bei empfindlicher Haut zu Irritationen führt. Schließlich lässt sich eine derartige Zubereitung nachteilig nicht mit anderen natürlichen ätherischen Ölen, Duftstoffen oder hautpflegenden Stoffen mischen.

Aus der BE 100 73 11 A6 ist ein natürliches Deodorant aus Kaliumalaun bekannt, dessen Benutzbarkeit durch einen Anteil von 0,7 % Xanthan-Gummi gesteigert werden kann. Die Zusammenfassung der Datenbank CA zur JP 56 046436 B4 offenbart eine Aerosol-Zusammensetzung, die durch Xanthan Gummi stabilisiert wird. Die US 2001/0006653 A1 beschreibt eine wässrige Zubereitung mit Carrageenanen, die Säuregruppen und Sulfatgruppen enthalten.

Aus der Zusammenfassung der Datenbank WPI zur JP 63 132661 A ist ein Deodorant bekannt, welches Alaun, eine Kupfer-Komponente, eine organische Säure, eine Ascorbinsäure-Komponente und optional Wasser enthält. Eine typische Zusammensetzung umfasst 100 Gew.-Teile Alaun, 0,02 - 50 Gew.-Teile Kupfer-Komponente, 5 - 100 Gew.-Teile organische Säure und 5 - 100 Gew.-Teile Ascorbinsäure-Komponente.

Der Erfindung liegt daher die Aufgabe zugrunde, ein Verfahren zur Herstellung einer desodorierenden Zubereitung der eingangs genannten Art bereitzustellen, welche nicht zum Auskristallisieren neigt. Die mittels eines solchen Verfahrens hergestellte Zubereitung soll sich außerdem mit natürlichen ätherischen Ölen, Duftstoffen oder hautpflegenden Stoffen mischen lassen.

Diese Aufgabe wird durch ein Verfahren zur Herstellung einer desodorierenden Zubereitung durch die Merkmale des Anspruchs 1 gelöst. Weiterbildungen und vorteilhafte Ausgestaltungen der Erfindung ergeben sich aus den Unteransprüchen 2 bis 9.

Erfindungsgemäß wird die Aufgabe durch ein Verfahren zur Herstellung einer desodorierenden Zubereitung gelöst, bei dem
(a) eine wässrige Lösung eines sauren Doppelsalzes der Formel

   M^{I}Al (SO₄)₂·12 H₂O (I)

   in der M^{I} ein Alkali- oder Ammonium-Ion ist, oder eine wässrige Lösung eines Gemisches verschiedener Doppelsalze der unter die allgemeine Formel (I) fallenden Gruppe, oder eine wässrige Lösung eines Gemisches der einfachen Salze, aus denen sich die Doppelsalze der unter die allgemeine Formel (I) fallenden Gruppe zusammensetzen, mit
(b) wenigstens einem Polysaccharid umgesetzt wird, welches aus einer Kette β-1,4-gebundener Glucose mit Seitenketten besteht, wobei die Seitenketten wenigstens eine Carboxylgruppe aufweisen.

Erfindungsgemäß wird das Polysacharid in Mengen von 0,1 bis 0,5 Gew.-%, besonders bevorzugt in Mengen von 0,1 bis 0,3 Gew.-% mit dieser vorzugsweise gesättigten Lösung vermischt.

Auf diese Weise werden Zubereitungen erhalten, die direkt mit einem Deo-Zerstäuber oder Deo-Roll-on appliziert werden können.

Die erfindungsgemäß hergestellte desodorierende Zubereitung ist gelartig und gegen Erhitzen, Gefrieren und milde Oxidation außerordentlich stabil. Die Zubereitung ist durchscheinend, leicht opak und flockt nicht aus. Es tritt keine Phasentrennung auf.

Es hat sich gezeigt, dass bei Verwendung eines Deo-Zerstäubers zum Aufsprühen der erfindungsgemäß hergestellten Zubereitung auf die Haut die in der Zubereitung enthaltenen Salze nicht in der Düse auskristallisieren. Dies ist darauf zurückzuführen, dass beim erfindungsgemäßen Umsetzen der Komponenten (a) und (b) ein Gelkomplex entsteht, der nicht zur Kristallisation neigt.

Darüber hinaus hat sich bei Tests an Probanden überraschenderweise herausgestellt, dass die erfindungsgemäß hergestellte Zubereitung eine bessere desodorierende Wirkung und eine bessere Hautverträglichkeit aufweist als die reine wässrige Lösung eines sauren Doppelsalzes der Formel

M^{I}Al(SO₄)₂ · 12 H₂O (I)

in der M^{I} ein Alkali- oder Ammonium-Ion ist, oder als die reine wässrige Lösung eines Gemisches verschiedener Doppelsalze der unter die allgemeine Formel (I) fallenden Gruppe, oder als die reine wässrige Lösung eines Gemisches der einfachen Salze, aus denen sich die Doppelsalze der unter die allgemeine Formel (I) fallenden Gruppe zusammensetzen.

Dies ist offensichtlich auf die Ausbildung eines neuen Wirkstoffkomplexes zurückzuführen, der dadurch entsteht, dass bei der Umsetzung von (a) mit (b) eine Veresterung der Hydroxylgruppe am C₆-Atom zumindest einiger Glucose-Einheiten mit Schwefelsäure erfolgt, wobei die hierbei gebildeten, negativ geladenen Sulfatgruppen sowie die in den Seitenketten vorhanden negativ geladen Carboxylgruppen eine zwischenmolekulare Anziehungskraft zu den positiv geladenen Aluminiumionen sowie zu den positiv geladenen M^{I}-Ionen ausüben. Der hierbei gebildete Gelkomplex verhindert nicht nur ein Auskristallisieren des gelösten Salzes, sondern führt auch zur Verbesserung der desodorierenden Wirkung der Zubereitung.

Die erfindungsgemäß hergestellte Zubereitung ist frei von jeglichen hautbelastenden Stoffen, wie Alkohol, synthetischen Zusätzen, insbesondere synthetischen Duft- und Konservierungsstoffen.

Vorteilhafterweise erfüllt eine derartig hergestellte Zubereitung auch die vom Bundesverband deutscher Industrie- und Handelsunternehmen (BDIH) in der Richtlinie "Kontrollierte Naturkosmetik" aufgeführten Anforderungen. Gemäß dieser Richtlinie dürfen nur natürliche Substanzen, nämlich solche, die eine gemeinsame Evolution mit den Menschen durchlaufen haben, verwendet werden. Dadurch liegt nur ein geringes toxikologisches Risikopotential vor. Insbesondere dürfen keine ethoxilierten Rohstoffe, Silikone, Paraffine oder andere Erdölprodukte verwendet werden.

Eine Weiterbildung des erfindungsgemäßen Verfahrens sieht vor, dass das Vermischen durch Rühren mit hoher Scherkraft bei Temperaturen von nicht mehr als 30 °C, vorzugsweise nicht mehr als 26 °C erfolgt, bis vollständige Lösung oder Quellung des Polysaccharids eingetreten ist.

Hierdurch wird eine stabile desodorierende Zubereitung mit langanhaltender Wirksamkeit und ausgezeichneter Hautverträglichkeit erhalten.

Vorzugsweise wird als wässrige Lösung (a) eine bei einer Temperatur zwischen 15°C und 25 °C, vorzugsweise bei 18 °C gesättigte Lösung verwendet.

Eine vorteilhafte Ausgestaltung der Erfindung sieht vor, dass als Polysaccharid Xanthan verwendet wird.

Xanthan ist ein mikrobielles anionisches Heteropolysaccharid. Es wird von Xanthomonas campestris und einigen anderen Species unter aeroben Bedingungen produziert. Xanthan wird aus einer Kette mit β-1,4-gebundener Glucose (Cellulose) mit Seitenketten gebildet. Die Struktur der Untergruppen ("repeated units") besteht aus Glucose, Mannose, Glucuronsäure, Acetat und Pyruvat. Xanthan ist vorteilhaft in Wasser gut löslich.

Die mit Xanthan als Polysaccharid hergestellte desodorierende Zubereitung zeichnet sich durch eine besonders gute desodorierende Wirkung und Hautverträglichkeit aus. Die Zubereitung ruft ein besonders angenehmes und geschmeidiges Hautgefühl hervor. Insbesondere ist die Zubereitung außerordentlich stabil gegen Hitze oder Kälte und kann daher auch über längere Zeit gelagert oder transportiert werden.

Die besonders gute desodorierende Wirkung und Hautverträglichkeit ist auf die Ausbildung eines neuen Wirkstoffkomplexes zurückzuführen, der dadurch entsteht, dass beim Umsetzen der wässrigen Lösung (a) mit Xanthan eine Veresterung der Hydroxylgruppe am C₆-Atom zumindest einiger Glucose-Einheiten mit Schwefelsäure erfolgt, wobei die hierbei gebildeten, negativ geladenen Sulfatgruppen sowie die in den Seitenketten vorhanden negativ geladen Carboxylgruppen eine zwischenmolekulare Anziehungskraft zu den positiv geladenen Aluminiumionen sowie zu den positiv geladenen M^{I}-Ionen ausüben. Nachfolgend ist die Strukturformel des genannten Wirkstoffkomplexes aufgeführt:

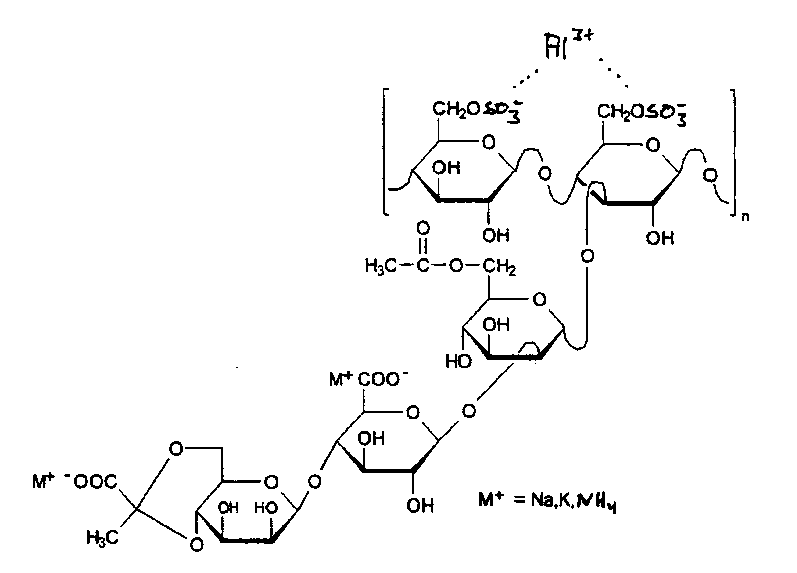

Eine Weiterbildung des erfindungsgemäßen Verfahrens sieht vor, dass das verwendete Xanthan eine Partikelgröße bis zu 100 µm, vorzugsweise bis zu 70 µm aufweist. Es hat sich herausgestellt, dass diese Partikelgrößen eine homogene Verteilung der Zubereitung und damit die Ausbildung des genannten Wirkstoffkomplexes fördern. Es wird eine Zubereitung mit guter Transparenz erhalten.

Weiterhin ist vorgesehen, dass der erfindungsgemäß hergestellten Zubereitung wenigstens eine organische Säure und/oder deren Salz, insbesondere Äpfelsäure und/oder deren Salz, Weinsäure und/oder deren Salz, Fumarsäure und/oder deren Salz, Gluconsäure und/oder deren Salz, Citronensäure und/oder deren Salz, vorzugsweise aber Milchsäure und/oder Natriumlactat, zugemischt wird. Vorzugsweise wird die organische Säure und/oder deren Salz in Mengen von je 0,1 bis 20 Gew.-% zugemischt. Es hat sich gezeigt, dass dadurch eine verbesserte desodorierende Wirkung erzielt wird.

Außerdem ist vorgesehen, dass wenigstens ein ätherisches Öl oder Duftstoff, insbesondere Rosenholzöl, Grapefruitöl, Geraniumöl, Menthol oder Kampfer, zugemischt wird. Vorzugsweise wird das ätherische Öl oder Duftstoff in Mengen von 0,01 bis 5 Gew.-% zugemischt. Die ätherischen Öle dienen einerseits als Duftstoffe, andererseits zeichnen sie sich durch ihre natürliche desinfizierende Wirkung aus, so dass die desodorierende und desinfizierende Wirkung der Zubereitung verbessert wird.

Dadurch, dass bei dem erfindungsgemäßen Verfahren überraschenderweise ein neuer desodorierender Wirkstoffkomplex gefunden wurde, bezieht sich die Erfindung außerdem auf eine desodorierende Zubereitung gemäß den Merkmalen des Anspruchs 10, durch die ein neuer desodorierender Wirkstoffkomplex bereitgestellt wird. Weiterbildungen und vorteilhafte Ausgestaltungen der erfindungsgemäßen Zubereitung ergeben sich aus den Unteransprüchen 11 bis 13.

Erfindungsgemäß besteht die desodorierende Zubereitung aus einer wässrigen Lösung einer sauren Aluminiumverbindung und wenigstens einem Polysaccharid, welches aus einer Kette β-1,4-gebundener Glucose mit Seitenketten besteht, die jeweils wenigstens eine Carboxylgruppe aufweisen, wobei die Hydroxylgruppe am C₆-Atom mindestens einiger Glucose-Einheiten mit Schwefelsäure verestert ist. Vorzugsweise ist die Carboxylgruppe Teil einer Pyruvat-Einheit der Seitenkette.

Wie bereits beschrieben, zeichnet sich eine derartige Zubereitung durch eine verbesserte desodorierende Wirkung und eine verbesserte Hautverträglichkeit aus.

Vorzugsweise ist die saure Aluminiumverbindung ein Doppelsalz der Formel

M^{I}Al (SO₄)₂·12 H₂O (I)

in der M^{I} ein Alkali- oder Ammonium-Ion ist, oder ein Gemisch verschiedener Doppelsalze der unter die allgemeine Formel (I) fallenden Gruppe, oder ein Gemisch der einfachen Salze, aus denen sich die Doppelsalze der unter die allgemeine Formel (I) fallenden Gruppe zusammensetzen.

Es ist vorgesehen, dass die Zubereitung
a) 90 bis 97 Gew.-%, vorzugsweise 95,5 bis 96,5 Gew.-% Wasser,
b) 2 bis 9 Gew.-%, vorzugsweise 3 bis 4 Gew.-% Aluminiumverbindung und
c) 0,05 bis 1 Gew.-%, vorzugsweise 0,1 bis 0,3 Gew.-% Polysaccharid umfasst.

Eine Weiterbildung sieht vor, dass das Polysaccharid Xanthan ist, wobei die Hydroxylgruppe am C₆-Atom wenigstens einiger seiner Glucose-Einheiten teilweise mit Schwefelsäure verestert ist.

Eine vorteilhafte Ausgestaltung der Erfindung sieht vor, dass dieser Zubereitung zusätzlich 0,1 bis 20 Gew.-% wenigstens einer organischen Säure und/oder deren Salz, vorzugsweise Milchsäure oder Natriumlactat, zumischbar sind.

Als organische Säure und/oder deren Salz eignen sich insbesondere Äpfelsäure und/oder deren Salz, Weinsäure und/oder deren Salz, Fumarsäure und/oder deren Salz, Gluconsäure und/oder deren Salz sowie Citronensäure und/oder deren Salz.

In einer Weiterbildung der Erfindung sind der Zubereitung zusätzlich 0,01 bis 5 Gew.-% wenigstens eines ätherisches Öls oder Duftstoffs, insbesondere Rosenholzöl, Grapefruitöl, Geraniumöl, Menthol oder Kampfer, zumischbar.

Die Erfindung betrifft auch die Verwendung der nach einem Verfahren gemäß einem der Ansprüche 1 bis 9 hergestellten Zubereitung oder der Zubereitung nach einem der Ansprüche 10 bis 13 als desodorierender Wirkstoff in Körperpflegemitteln, insbesondere in Deo-Zerstäubern, Aerosol-Sprays, Roll-ons, Duschgels, Fußsprays und Fußbädern.

### Beispiele

Herstellung einer desodorierenden Zubereitung für einen Deo-Zerstäuber:

Zunächst wird eine bei 18 °C gesättigte, wässrige Lösung eines mehrfach kristallisierten Ammoniumalauns hergestellt und in einen verschließbaren Druckbehälter eingebracht.

Die Herstellung von Ammoniumalaun erfolgt durch Zusammengeben der wässrigen Lösungen von (NH₄)₂SO₄ und Al₂(SO₄)₃ und anschließendem Auskristallisieren. Der Ausgangsstoff Al₂(SO₄)₃ ist aus H₂SO₄ und Al(OH)₃ herstellbar. (NH₄)₂SO₄ ist im Handel beispielsweise erhältlich von der BASF AG, Ludwigshafen, Deutschland. H₂SO₄ ist im Handel beispielsweise erhältlich von der Bayer AG, Leverkusen, Deutschland. Al(OH)₃ ist im Handel beispielsweise erhältlich von der NABALTEC GmbH, Schwandorf, Deutschland.

Die gesättigte, wässrige Lösung wird in dem geschlossenen Druckbehälter derart lange auf 80 °C erhitzt, bis die Lösung keimfrei ist. Anschließend wird die Lösung auf ca. 20 °C abgekühlt und mit 0,1 Gew.-% Xanthan versetzt. Xanthan ist im Handel beispielsweise erhältlich von der Jungbunzlauer Int. AG, Basel, Schweiz. Nach dem Verschließen des Druckbehälters wird ein Vakuum von etwa -850 mbar eingestellt. Daraufhin wird die Mischung durch Rühren mit hoher Scherkraft vermischt, bis eine vollständige Lösung des Xanthans eingetreten ist, wobei die sich ergebende Temperaturerhöhung 30 °C nicht überschreiten sollte. Anschließend wird die Zubereitung auf etwa 18 °C abgekühlt, wobei das eingestellte Vakuum auf -950 mbar nachgeführt wird. Hier wird die fertige desodorierende Zubereitung entgast, so dass ein qualitativ hochwertiges, stabiles Produkt entsteht. Die desodorierende Zubereitung ist ohne weiteres direkt in einem Deo-Zerstäuber verwendbar.

Herstellung einer desodorierenden Zubereitung für einen Deo-Roll-on:

Die Herstellung erfolgt wie die Herstellung einer desodorierenden Zubereitung für einen Deo-Zerstäuber, jedoch wird die gesättigte Lösung nicht mit 0,1 Gew.-%, sondern mit 0,3 Gew.-% Xanthan versetzt. Die desodorierende Zubereitung ist ohne weiteres direkt in einem Deo-Roll-on verwendbar.

Die Menge jedes Inhaltsstoffs wird in der vorliegenden Patentbeschreibung und den vorliegenden Patentansprüchen ausgedrückt als Gew.-% bezogen auf die Gesamtzusammensetzung. Unter Gew.-% der Feststoffe wird immer Trockengew.-% verstanden.

## Patentansprüche

1. Verfahren zur Herstellung einer desodorierenden Zubereitung, wobei
(a) eine wässrige Lösung eines sauren Doppelsalzes der Formel
M^{I}Al(SO₄)₂·12 H₂O (I)
in der M^{I} ein Alkali- oder Ammonium-Ion ist, oder eine wässrige Lösung eines Gemisches verschiedener Doppelsalze der unter die allgemeine Formel (I) fallenden Gruppe, oder eine wässrige Lösung von Gemischen der einfachen Salze, aus denen sich die Doppelsalze der unter die allgemeine Formel (I) fallenden Gruppe zusammensetzen, mit
(b) wenigstens einem Polysaccharid umgesetzt wird, welches aus einer Kette β-1,4-gebundener Glucose mit Seitenketten besteht, wobei die Seitenketten wenigstens eine Carboxylgruppe aufweise, **dadurch gekennzeichnet, dass**
das Polysaccharid in Mengen von 0,1 bis 0,5 Gew.-%, besonders bevorzugt in Mengen von 0,1 bis 0,3 Gew.-% eingesetzt wird.

2. Verfahren nach Anspruch 1, **dadurch gekennzeichnet, dass** die Umsetzung durch Rühren mit hoher Scherkraft bei Temperaturen von nicht mehr als 30 °C, vorzugsweise nicht mehr als 26 °C erfolgt, bis vollständige Lösung oder Quellung des Polysaccharids eingetreten ist.

3. Verfahren nach Anspruch 1 oder 2, **dadurch gekennzeichnet, dass** als Polysaccharid Xanthan verwendet wird.

4. Verfahren nach Anspruch 3, **dadurch gekennzeichnet, dass** das verwendete Xanthan eine Partikelgröße bis zu 100 µm, vorzugsweise bis zu 70 µm aufweist.

5. Verfahren nach einem der Ansprüche 1 bis 4, **dadurch gekennzeichnet, dass** der nach einem der Ansprüche 1 bis 4 hergestellten Zubereitung wenigstens eine organische Säure und/oder deren Salz, vorzugsweise Milchsäure und/oder Natriumlactat, zugemischt wird.

6. Verfahren nach einem der Ansprüche 1 bis 5, **dadurch gekennzeichnet, dass** als wässrige Lösung (a) eine bei einer Temperatur zwischen 15 °C und 25 °C, vorzugsweise bei 18 °C gesättigte Lösung verwendet wird.

7. Verfahren nach einem der Ansprüche 5 bis 6, **dadurch gekennzeichnet, dass** die organische Säure und/oder deren Salz in Mengen von je 0,1 bis 20 Gew.-% zugemischt wird.

8. Verfahren nach einem der Ansprüche 1 bis 7, **dadurch gekennzeichnet, dass** wenigstens ein ätherisches Öl oder Duftstoff, insbesondere Rosenholzöl, Grapefruitöl, Geraniumöl, Menthol oder Kampfer, zugemischt wird.

9. Verfahren nach Anspruch 8, **dadurch gekennzeichnet, dass** das ätherische Öl oder der Duftstoff in Mengen von 0,01 bis 5 Gew.-% zugemischt wird.

10. Desodorierende Zubereitung aus
a) einer wässrigen Lösung eines saures Doppelsalz der Formel
M^{I}Al(SO₄)₂·12 H₂O (I)
in der M' ein Alkali- oder Ammonium-lon ist, oder ein Gemisch verschiedener Doppelsalze der unter die allgemeine Formel (I) fallenden Gruppe, oder ein Gemisch der einfachen Salze, aus denen sich die Doppelsalze der unter die allgemeine Formel (I) fallenden Gruppe zusammensetzen, und
b) wenigstens einem Polysaccharid, welches aus einer Kette β-1,4-gebundener Glucose mit Seitenketten besteht, die jeweils wenigstens eine Carboxylgruppe aufweisen, wobei die Hydroxylgruppe am C₆-Atom mindestens einiger Glucose-Einheiten mit Schwefelsäure verestert ist, **dadurch gekennzeichnet, dass** die Zubereitung
i) 90 bis 97 Gew.-%, vorzugsweise 95,5 bis 96,5 Gew.-% Wasser,
ii) 2 bis 9 Gew.-%, vorzugsweise 3 bis 4 Gew.-% Doppelsalz und
iii) 0,05 bis 1 Gew.-%, vorzugsweise 0,1 bis 0,3 Gew.-% Polysaccharid umfasst.

11. Desodorierende Zubereitung nach Anspruch 10, **dadurch gekennzeichnet, dass** das Polysaccharid Xanthan ist, wobei die Hydroxylgruppe am C₆-Atom wenigstens einiger seiner Glucose-Einheiten mit Schwefelsäure verestert ist.

12. Desodorierende Zubereitung nach Anspruch 10 oder 11, **dadurch gekennzeichnet, dass** der Zubereitung nach Anspruch 10 oder 11 zusätzlich je 0,1 bis 20 Gew.-% wenigstens einer organischen Säure und/oder deren Salz, vorzugsweise Milchsäure und/oder Natriumlactat, zumischbar sind.

13. Desodorierende Zubereitung nach einem der Ansprüche 10 bis 12, **dadurch gekennzeichnet, dass** der Zubereitung nach einem der Ansprüche 10 bis 12 zusätzlich 0,01 bis 5 Gew.-% wenigstens eines ätherisches Öls oder Duftstoffs, insbesondere Rosenholzöl, Grapefruitöl, Geraniumöl, Menthol oder Kampfer, zumischbar sind.

14. Verwendung der nach einem Verfahren gemäß einem der Ansprüche 1 bis 9 hergestellten Zubereitung oder der Zubereitung nach einem der Ansprüche 10 bis 13 als desodorierender Wirkstoff in Körperpflegemitteln, insbesondere in Pumpsprays, Aerosolsprays, Roll-ons, Duschgels, Fußsprays und Fußbädern.

## Claims

1. Process for producing a deodorant preparation, wherein
(a) an aqueous solution of an acid double salt of formula
M^{I}Al (SO₄)₂ · 12 H₂O (I),
in which M^{I} is an alkali metal ion or ammonium ion, or an aqueous solution of a mixture of different double salts from the group which falls under general formula (I), or an aqueous solution of mixtures of the single salts from which the double salts from the group which falls under general formula (I) are composed, is reacted with
(b) at least one polysaccharide which consists of a chain of β-1,4-bonded glucose with side chains, wherein the side chains comprise at least one carboxyl group,
**characterised in that**
the polysaccharide is used in amounts of 0.1 to 0.5% by weight, particularly preferably in amounts of 0.1 to 0.3% by weight.

2. Process according to Claim 1, **characterised in that** the reaction is carried out by stirring with high shear force at temperatures of no more than 30°C, preferably no more than 26°C, until complete dissolution or swelling of the polysaccharide is obtained.

3. Process according to Claim 1 or 2, **characterised in that** xanthan is used as the polysaccharide.

4. Process according to Claim 3, **characterised in that** the xanthan used has a particle size of up to 100 µm, preferably up to 70 µm.

5. Process according to one of Claims 1 to 4, **characterised in that** at least one organic acid and/or the salt thereof, preferably lactic acid and/or sodium lactate, is added to the preparation produced according to one of Claims 1 to 4.

6. Process according to one of Claims 1 to 5, **characterised in that** a solution which is saturated at a temperature between 15°C and 25°C, preferably at 18°C, is used as the aqueous solution (a).

7. Process according to one of Claims 5 to 6, **characterised in that** the organic acid and/or the salt thereof is added in amounts of in each case 0.1 to 20% by weight.

8. Process according to one of Claims 1 to 7, **characterised in that** at least one essential oil or fragrance, in particular rosewood oil, grapefruit oil, geranium oil, menthol or camphor, is added.

9. Process according to Claim 8, **characterised in that** the essential oil or fragrance is added in amounts of 0.01 to 5% by weight.

10. Deodorant preparation composed of
(a) an aqueous solution of an acid double salt of formula
M^{I}Al(SO₄)₂· 12 H₂O (I) ,
in which M^{I} is an alkali metal ion or ammonium ion, or a mixture of different double salts from the group which falls under general formula (I), or a mixture of the single salts from which the double salts from the group which falls under general formula (I) are composed, and
(b) at least one polysaccharide which consists of a chain of β-1,4-bonded glucose with side chains, which side chains in each case comprise at least one carboxyl group, wherein the hydroxyl group on the C₆ atom of at least some glucose units is esterified with sulphuric acid, **characterised in that** the preparation comprises
i) 90 to 97% by weight, preferably 95.5 to 96.5% by weight, of water,
ii) 2 to 9% by weight, preferably 3 to 4% by weight, of double salt, and
iii) 0.05 to 1% by weight, preferably 0.1 to 0.3% by weight, of polysaccharide.

11. Deodorant preparation according to Claim 10, **characterised in that** the polysaccharide is xanthan, wherein the hydroxyl group on the C₆ atom of at least some of its glucose units is esterified with sulphuric acid.

12. Deodorant preparation according to Claim 10 or 11, **characterised in that** additionally in each case 0.1 to 20% by weight of at least one organic acid and/or the salt thereof, preferably lactic acid and/or sodium lactate, can be added to the preparation according to Claim 10 or 11.

13. Deodorant preparation according to one of Claims 10 to 12, **characterised in that** additionally 0.01 to 5% by weight of at least one essential oil or fragrance, in particular rosewood oil, grapefruit oil, geranium oil, menthol or camphor, can be added to the preparation according to one of Claims 10 to 12.

14. Use of the preparation produced by a process according to one of Claims 1 to 9 or of the preparation according to one of Claims 10 to 13 as a deodorising active substance in body care products, in particular in pump sprays, aerosol sprays, roll-ons, shower gels, foot sprays and foot soaks.

## Revendications

1. Procédé de fabrication d'une préparation désodorisante, suivant lequel (a) une solution aqueuse d'un sel double de la formule
M^{I}Al(So₄)₂ · H₂O (I),
où M₁ est un ion d'alcali ou d'ammonium ou une solution aqueuse d'un mélange de différents sels doubles du groupe appartenant à la formule générale (I) ou une solution aqueuse de mélanges de sels simples qui composent les sels doubles du groupe appartenant à la formule générale (I), est transformée avec
(b) au moins un polysaccharide, qui est composé d'une chaîne β-1,4- de glucoses agglomérés avec des chaînes latérales, les chaînes latérales présentant au moins un groupe carboxyle, **caractérisé en ce que** le polysaccharide est utilisé dans des quantités allant de 0,1 à 0,5% en poids, et de préférence dans des quantités allant de 0,1 à 0,3% en poids.

2. Procédé suivant la revendication 1, **caractérisé en ce que** la transformation a lieu par agitation avec une force de cisaillement élevée à des températures inférieures à 30°C et de préférence à 26°C, jusqu'à dissolution ou gonflement intégral du polysaccharide.

3. Procédé suivant la revendication 1 ou 2 **caractérisé en ce que** de la xanthane est utilisé comme polysaccharide.

4. Procédé suivant la revendication 3, **caractérisé en ce que** la xanthane utilisée présente une grosseur de particule jusqu'à 100 µm, de préférence jusqu'à 70 µm.

5. Procédé suivant une des revendications 1 à 4, **caractérisé en ce qu'**au moins un acide organique et/ou le sel de celui-ci, de préférence de l'acide lactique et/ou du lactate de sodium sont mélangés à la préparation fabriquée suivant une des revendications 1 à 4.

6. Procédé suivant une des revendications 1 à 5, **caractérisé en ce que** comme solution aqueuse (a) est utilisée une solution saturée à une température comprise entre 15°C et 25°C et de préférence à une température de 18°C.

7. Procédé suivant une des revendications 5 ou 6, **caractérisé en ce que** l'acide organique et/ou le sel de celui-ci est additionné par mélange dans des quantités allant de 0,1 à 20% en poids respectivement.

8. Procédé suivant une des revendications 1 à 7, **caractérisé en ce qu'**au moins une huile essentielle ou une matière odoriférante, en particulier de l'essence de bois de rose, de l'essence de pamplemousse, de l'essence de géranium, du menthol ou du camphre sont additionnés par mélange.

9. Procédé suivant la revendication 8, **caractérisé en ce que** l'huile essentielle ou la matière odoriférante est additionnée par mélange dans des quantités allant de 0,01 à 5 % en poids.

10. Préparation désodorisante composée
(a) d'une solution aqueuse d'un sel double acide de la formule
M¹AI(SO₄)₂ · 12 H₂O (I),
où M' est un ion d'alcali ou d'ammonium ou un mélange de différents sels doubles du groupe appartenant à la formule générale (I) ou un mélange des sels simples qui composent les sels doubles du groupe appartenant à la formule générale (I) et
(b) au moins un polysaccharide qui est constitué d'une chaîne β-1,4 de glucoses agglomérés avec chaînes latérales qui présentent respectivement au moins un groupe carboxyle, le groupe hydroxyle étant, sur l'atome C6 de quelques unités de glucose au moins, estérifié avec de l'acide sulfurique, **caractérisé en ce que** la préparation comprend
i) de 90 à 97 % en poids, de préférence de 95,5 à 96,5% en poids d'eau,
ii) de 2 à 9% en poids, de préférence de 3 à 4% en poids de sel double et
iii) de 0,05 à 1 % en poids, de préférence de 0,1 à 9,3 % en poids de polysaccharide.

11. Préparation désodorisante suivant la revendication 10, **caractérisée en ce que** le polysaccharide est de la xanthane, le groupe hydroxyle étant, sur l'atome C6 de quelques-unes au moins de ses unités de glucose, estérifié avec de l'acide sulfurique.

12. Préparation désodorisante suivant la revendication 10 ou 11, **caractérisée en ce que**, en plus, respectivement 0,1 à 20 % en poids au moins d'un acide organique et/ou de son sel, de préférence de l'acide lactique et/ou du lactate de sodium peuvent être additionnés par mélange.

13. Préparation désodorisante suivant une des revendications 10 à 12, **caractérisée en ce que,** en plus, 0,01 à 5 % en poids au moins d'une huile essentielle ou d'une matière odoriférante, en particulier, de l'essence de bois de rose, de l'essence de géranium, du menthol ou du camphre peuvent être additionnés par mélange.

14. Utilisation de la préparation réalisée suivant un procédé selon une des revendications 1 à 9 ou de la préparation suivant une des revendications 10 à 13 comme agent désodorisant dans des produits de soins corporels, en particulier dans des atomiseurs à pompe, des atomiseurs aérosols, des roll-ons, des gels douche, des déodorants pour pieds en vaporisateur et des bains de pieds.
